# EUROPEAN PATENT APPLICATION

(11) **EP 2 361 623 A1**
(43) Date of publication of application: **31.08.2011**
(21) Application number: 10001299.6
(22) Date of filing: 09.02.2010
(51) Int. Cl.: A61K 31/353, A61K 38/01, A61P 1/12

(54) **Composition comprising a polyphenol salt and corresponding use**

(71) Applicant: Novintethical Pharma, SAGL, 6915 Pambio-Noranco Lugano (CH)
(72) Inventor: Alonso Cohen, Miguel Angel, 08034 Barcelona (ES); Durbán Reguera, Rafael, 04001 Almeria (ES)
(74) Representative: Curell Aguilà, Marcelino

(57) **Abstract**

Composition comprising a polyphenol salt, with a molecular weight of between 500 and 9000, and gelatine. The salt has pharmacological properties such as protecting the mucous in the digestive tract, as an astringent and as an anti-diarrhoea product. It is suitable for treating infant dysenteriform enterocollitis and exasperated intestinal flora. It is suitable for use both in adults and children and weening infants. Moreover, the polyphenolic component adsorbs the albuminoidal and mucilaginous substances, thereby preventing their harmful action from irritating the intestinal mucous and, also, the gelatine mechanically protects the inflammed mucous against acids and alkalines, The gelatine makes it possible to reduce the stomach irritation that could be caused by the polyphenol in patients who are sensitive to it. Thanks to the proline content in the gelatine, the systemic absorption of the polyphenol is inhibited.

## Description

### Field of the invention

The invention relates to a composition comprising a polyphenol salt. The invention also relates to the use of a composition comprising a polyphenol salt.

### State of the art

The use of various products to protect the digestive mucous is known. So, for example, the use of omeprazol is known for treating, for example, dyspepsia, peptic ulcers, gastro-oesophageal reflux diseases and the Zollinger-Ellison syndrome. Omeprazol inhibits the proton pump in the stomach. Other proton pump inhibitors are, for example, lansoprazol, esomeprazol, pantoprazol and rabeprazol. Although these compounds have few side effects, they are not totally free of side effects, and these include: headache, nausea, diarrhoea, stomach pain, fatigue and vertigo.

### Disclosure of the invention

The aim of the invention is to overcome these drawbacks. This aim is achieved by means of a composition comprising a polyphenol salt, with a molecular weight of between 500 and 9000, and gelatine. Preferably the polyphenol has a molecular weight of between 500 and 3000.

In fact, this salt has pharmacological properties, such as protecting the mucous in the digestive tract, and also as an astringent and anti-diarrhoea product. It is also suitable for treating infant dysenteriform enterocollitis and exasperated intestinal flora. It is suitable for use both in adults and children and weening infants, Moreover, the polyphenolic component, which preferably is a tannate and very preferably tannic acid, adsorbs the albuminoidal and mucilaginous substances, thereby preventing their harmful action that irritates the intestinal mucous and, on the other hand, the gelatine mechanically protects the inflammed mucous from acids and alkalines. Also, the gelatine makes it possible to reduce stomach irritation that could be caused by the polyphenol in patients who are sensitive to it. In addition, the gelatine is a protein substance containing proline and, thanks to the proline content, it inhibits the systemic absorption of the tannins, which increases the composition's safety and tolerance, and this is particularly interesting considering that its use frequently means repeatedly taking various doses.

In addition, the polyphenol salt with gelatine has the advantage that it does not break down in the presence of the acidic pH in the stomach, and instead it only breaks down in a base medium, like the one in the intestine.

Preferably the composition only contains the polyphenol salt with gelatine. In fact, this salt does not require any other type of additive or excipient, and this facilitates its preparation and prevents any kind of side effect caused by said additional additives. Preferably the dosage is in sachets or capsules containing between 250 and 500 mg of the salt. Advantageously, the sachets contain 250 mg of the salt, and the capsules contain 500 mg of the salt.

The aim of the invention is also the use of a polyphenol derivative for preparing a medicine for protecting the digestive mucous, where preferably the polyphenol derivative has a molecular weight of between 500 and 9000, and very preferably of between 500 and 3000. Advantageously the polyphenol derivative is a salt of polyphenol and gelatine, and preferably the polyphenol is a tannate (and advantageously, it is tannic acid).

### Detailed description of some embodiments of the invention

### Production method:

An example of the production process is described below, which includes the following stages:
Raw Material

The raw material used to produce gelatine tannate is as folows.
1. Tannic acid, CAS number 121-79-9, according to European pharmacopoeia, minimum richness 97 %, extraction method, in ether, water or alcohol.
2. A type, 18 mesh Gelatine, 60 - 80 Bloom. Pharmaceutical quality. Supplier Junca
3. Water. Sanitary quality.

Stage 1: dissolve 11 kg of gelatine in a tank that has a capacity of 5001, with hot water, at 45°C. The gelatine is added to the water slowly, while stirring. With a 18 mesh gelatine, the dissolution time is almost immediate. It is advantageous to provide a dissolution time of about 10-12 minutes. At the same time, in another tank that has a capacity of 10001, dissolve 16 kg of tannic acid in water at 22°C, while stirring (generally, the water can be at room temperature). The handling variables in this stage are the temperature of the water where the reagents are dissolved and the volume of water to be used.

Generally, the proportions of water according to the amount of tannic acid are 25 litres of water for every kg of tannic acid, and the proportions of water according to the amount of gelatine are 10 litres of water for every kg of gelatine.

Stage 2: once both reagents have been dissolved, the gelatine dissolution is to be added over the tannic acid dissolution. Throughout both the dissolution and the reaction process, stirring is maintained continuously, using two blades driven by a-motor at 100 rpm.

The handling variables in this stage are the flow rate at which the gelatine enters the tank where the tannic acid is contained, and the stirring speed in the tank.

Stage 3: once the gelatine has been added, the stirring is stopped and it is necessary to wait for the reaction to finish, approximately 5 minutes. Let the solution precipitate in the tank for a period of 15 minutes. The handling variables are the temperature at which the reaction is produced and the time that it is left to precipitate. Optionally the precipitation time can be maintained for about 2 hours, and also cold water can be added.

Stage 4: after separating into two phases (water and gelatine tannate), remove the excess water remaining after the reaction that will contain part of the tannic acid used, owing to the excess acid used because of the need to work in an acidic medium.

Stage 5: add ice to the precipitate for cooling, before spinning it. The handling variable is the cooling temperature.

Stage 6: the precipitate is pumped into a discontinuous centrifuge that uses a hose pie. It is spinned for 5 minutes at 2100 rpm.

The handling variables are the spinning time and speed.

Stage 7: after spinning, the gelatine tannate is ground to thereby facilitate the drying thereof, which will take place in the following stage. Once ground, it is added to the drier. This stage is optional.

Stage 8: the drier is programmed to remove the water contained in the gelatine tannate. The drying program will be made up of several steps, each one with a certain temperature and duration.
- 10°C for 10 h
- 20°C for 6 h
- 33°C for 6h

Preferably the gelatine tannate, once dried, contains between 6% and 8% humidity, which is an optimum level for the subsequent methods.

Stage 9: once dried it is collected, weighed and ground. Finally, a sample is taken for Quality Control.

| Analysis | Results |
|---|---|
| Appearance | Earthy, yellowish white powder |
| Weight loss | 7,2 % |
| Ignition residue: | 0,33 % |
| Solubility | Insoluble in water and organic solvents |
| Presence of sulphites | 0,12 ppm |
| Arsenic | 0,06 ppm |
| Heavy metals | 1,5 ppm |
| **Identification reactions** | |
| IR Spectrophotometry | Corresponds to the reference substance |
| Colorimetric reaction with ferric chloride | Blue colouring |
| **Assessment** | |
| IR Spectrophotometry | 96,2 % |

### Clinical Assay:

A total of 20 children were selected, aged between 3 and 12 years, with signs of acute diarrhoea. They were given 250 mg of gelatine tannate in powder format, 6 times a day for 2 days.

### Effectiveness analysis:

The amount of liquid depositions per day was analysed. The results obtained (on 19 children) are shown in Table 1:

**Table 1**

| | Min. | Max. | Average | Error statistic. | Deviation statistic. |
|---|---|---|---|---|---|
| Depositions day 1 | 3 | 5 | 3,47 | 160 | 0,697 |
| Depositions day 2 | 2 | 4 | 2,47 | 0,140 | 0,612 |
| Depositions day 3 | 1 | 2 | 1,74 | 0,104 | 0,452 |

The difference between the values before starting the assay and the values after two days of the assay are significant (p<0,01). Therefore, it is concluded that the gelatine tannate significantly reduces the amount of liquid depositions.

The reduction in stomach pain was also analysed on the same subjects, and it was revealed that the gelatine tannate significantly reduces stomach pain (p<0,01).

None of the children showed any adverse side effects.

## Claims

1. Composition comprising a polyphenol salt, with a molecular weight of between 500 and 9000, and gelatine.

2. Composition according to claim 1, **characterized in that** said polyphenol has a molecular weight of between 500 and 3000.

3. Composition according to one of the claims 1 or 2, **characterized in that** it only contains said salt.

4. Composition according to any of the claims 1 to 3, **characterised in that** said polyphenol is a tannin.

5. Composition according to claim 4, **characterized in that** said tannin is tannic acid.

6. Use of a polyphenolic derivative for preparing a medicine for protecting the digestive mucous.

7. Use according to claim 6, **characterized in that** said polyphenolic derivative has a molecular weight of between 500 and 9000, preferably between 500 and 3000.

8. Use according to any of the claims 6 or 7, **characterized in that** said polyphenol derivative is a salt of polyphenol and gelatine.

9. Use according to claim 8. **characterized in that** said polyphenol is a tannin.

10. Use according to claim 9, **characterized in that** said tannin is tannic acid.
